# EUROPEAN PATENT SPECIFICATION

(11) **EP 0 215 907 B1**
(45) Date of publication and mention of the grant of the patent: **23.05.2001**
(21) Application number: 86902165.9
(22) Date of filing: 17.03.1986
(51) Int. Cl.: C12N 15/09, C12N 1/20, C12N 1/00, C12N 5/00

(54) **PARASITE-DERIVED RESISTANCE**
VOM PARASIT ABGELEITETER WIDERSTAND
RESISTANCE DERIVEE D'UN PARASITE

(30) Priority: 21.03.1985 US 714263
(43) Date of publication of application: 01.04.1987
(60) Divisional application: 91117193.2
(73) Proprietor: Johnston, Stephen, Ph.D., Dallas, TX 75235-8573 (US); CORNELL RESEARCH FOUNDATION, INC., Ithaca, N.Y. 14850 (US)
(72) Inventor: JOHNSTON, Stephen, A., Durham, NC 27701 (US); SANFORD, John, C., Geneva, NY 14456 (US)
(74) Representative: Giambrocono, Alfonso, Dr. Ing.
(86) International application number: US8600514
(87) International publication number: WO8605516

(56) References cited:
- Trends in Biochemical Science, vol. 10, no. 6, iss. June 1985, Nordströme Kurt, s. p. 232
- Journal of Cell Biology, vol. 101, pp. 1094-1099, iss. Sept. 1985, Harland, et al.
- Virology, vol. III, pp. 301-311, iss. 1981; Zimmer et al. s. pp. 301, 310
- Journal of Biological Chemistry, vol. 260, no. 16, iss. August 5, 1985, pp. 9085-9087 Ellison et al. s. entire document
- Science, vol. 229, pp. 234-352, iss. July 1985, Izant et al. s. entire document
- Nature, vol. 313, pp. 703-706, iss. 21 February 1985, Rosenberg et al. s. entire doc.
- Proceedings of the Japan Academy, vol. 59, Series B, pp. 33-4338, iss. 1983, Mizuno et al. s. p. 336
- Cell, vol. 42, pp. 129-138, iss. Augsut 1985, Kim et al. s. p. 136
- Proceedings of the National Academy of Science, vol. 82, pp. 144-148, iss. January 1985, Melton D.A. s. p. 148
- Trends in Genetics, vol. 1, no. 1, pp. 22-25, iss. January 1985, Weintraub et al. s. pp. 24-25
- Molecular and Cellular Biology, vol. 5, no. 9, p. 2341-2348, iss. Sept. 1985, Chang et al. s. entire doc.
- PROC. NATL. ACAD. SCI. USA, vol. 75, no. 1, January 1978, pp. 280-284; US P.C. ZAMECNIK et al.
- PROC. NATL. ACAD. SCI. USA, vol. 75, no. 1, January 1978, pp. 285-288, US M.L. STEPHENSON et al.
- PLANT MICROBE INTERACTIONS, 1984, pp. 420-429: Chapter 17; McMillan Inc. P. PALUKAITIS et al. p. 422, p. 425, p. 427
- J. THEOR. BIOL. vol. 113, no. 2, March 21, 1985, pp. 395-405, Acad. Press Inc., London, GB J.C. SANFORD et al.
- BIO/TECHNOLOGY, vol. 4, February 1986, p. 96, H. BIALY et al.
- BIOLOGICAL ABSTRACTS, vol. 80, no. 11, 1985, p. AB-363, ref.no. 94988; Philadelphia, USA; M.W. BEVAN et al. & EMBO, 14(8), 1921-1926, 1985
- Trends in Biochemical Science, Vol. 10, No. 6, issued June 1985; NORDSTROM, KURT "Antisense RNA" see page 232
- Journal of Cell Biology, Vol. 101 pp. 1094-1099 issued September 1985; HARLAND, et al, "Translation of mRNA Injected into Xenopus Oocytes is Specifically Inhibited by Antisense RNA" see Entire Document
- Virology, Vol. III pp 301-311 issued 1981; ZIMMER, et al, "Genome Distribution of Adenovirus Total and Self -Complementary Nuclear RNA at Early Times" see pages 301, 310
- Journal of Biological Chemistry, Vol. 260 No. 16, issued August 5, 1985, pages 9085-9087; ELLISON, et al; "Thermal Regulation of Beta-Galactosidase Synthesis using Antisense RNA Directed against the Coding Portion of the mRNA" see Entire Document
- Science, Vol. 229, pages 234-352, issued July 1985; IZANT, et al.; "Const Itutive and Conditional Suppression of Exogenous and Endogenous Genes by Anti-Sense RNA" see Entire Document
- Nature Vol. 313, pages 703-706, issued 21 February 1985, ROSENBERG, et al; "Production of Phenocopies by Kruppel Antisense RNA Injection into Drosophila Embryos" see Entire Document
- Proceedings of the Japan Academy Vol. 59 Series B, pages 33-4338, issued 1983, MIZUNO, et al; "Regulation of Gene Expression by a small RNA Transcript (micRNA) in Escherichia Coli K-12" see page 336 in Particular
- Cell Vol. 42, pp 129-138, issued August 1985; KIM, et al; "Stable Reduction of Thymidine Kinase Activity in Cells Expressing High Levels of Anti-Sense RNA" see page 136 in Particular
- Proceedings of the National Academy of Science Vol. 82 pp 144-148, issued January 1985, MELTON, D.A., "Injected Anti-Sense RNAs Specifically Block Messenger RNA Translation in Vivo" see page 148 in Particular
- Trends in Genetics, Vol. 1 No. 1 pp 22-25, issued January 1985; WEINTRAUB, et al; "Anti-Sense RNA as a Tool for Genetic Analysis" see pages 24-25 in Particular
- Molecular and Cellular Biology, Vol. 5 No. 9, page 2341-2348, issued September 1985; CHANG, et al; "Gene Expression from Both Intronless and Intron-Containing Rous Sarcoma Virus Clones is Specifically Inhibited by Anti-Sense RNA" see Entire Document
- Palukaitis, P. and Zaitlin, M. (1984). A model to explain the "cross-protecion" phenomenon shown by plant viruses and viroids. In: Plant-Microbe interactions. McMillan Inc. pp 420-429.
- SEQUEIRA, L.: "Cross protection and induced resistance: their potential for plant disease control", TRENDS IN BIOTECHNOLOGY (1984), , 01. March 1984, vol. 2, no. 2, pages 25 to 29

## Description

The present invention relates to methods of conferring resistance to parasites, such as viruses, to hosts of the parasite. More particular, this invention relates to parasite resistance obtained by genetic engineering of the host organism.

### Background Art

A potentially important application of genetic engineering technology is in the area of producing resistance to parasites. The proposals in the prior art that have been systematic and broadly applicable have centered on finding a gene conferring resistance within a strain of the host species or within a related species and transforming the gene into the genome of a susceptible host. This approach may prove effective but has several distinct disadvantages. Resistant forms of the host may not exist or may be very difficult to find for each new race of parasite which arises. Such resistance may be polygenic, making the cloning and transfer of the resistance genes difficult. Where resistance is encoded by a gene, there are commonly already strains of the parasite that have evolved virulence genes for overcoming such host-derived resistances in a gene-for-gene fashion (Flor 1971). Finally, the problem of identifying and isolating the resistance gene from within the large genome of the host will generally remain very difficult. An alternative strategy that addresses these problems is therefore needed.

There have also been proposals for and some work on using genes from organisms unrelated to either host or parasite, which serendipitously have gene products detrimental to a specific parasite. The gene coding for the endotoxin of Baccillus thuringiensis (which is toxic to lepidopterous insects) would be an example of this (Held et al., 1982). While this type of approach may prove useful in some specific cases, it clearly represents an opportunistic approach to the problem, as opposed to a systematic methodology that can be applied very broadly.

There already exist some examples of genes, gene derivatives, or gene products of a parasite that can produce a negative interaction with itself or a related genotype. Studies into the susceptibility of plants to infection by viruses have demonstrated that closely related plant viruses or different strains of the same virus will cross-protect a host organism (Hamilton, 1980). In other words, a plant infected by a first virus is often not subject to infection by a second strain of that virus or by a related virus. A similar phenomenon has been observed in animal viruses and has been termed intrinsic interference (Marcus and Carrier, 1967). From the point of view of parasite resistance of the type discussed herein, the key proteins involved in the intrinsic interference phenomenon are the viral replicase proteins (Marcus and Zuckerbraun, 1970). These same authors proposed that the replicase proteins of the primary infecting virus prevent the replication of the second virus by binding to its replicase attachment sites (Marcus and Zuckerbraun, 1969). A similar proposal has been put forth to explain cross-protection in plants (Gibbs, 1969). In a similar manner, experimenters working with an E. coli infected with bacteriaphage 434 have found that infected bacteria are immune to other phages (Lauer et al, 1981; Flashman, 1973; Roberts et al, 1979). Other workers have noticed that endogenous as well as experimentally introduced complementary oligonucleotides can interact with mRNA in a potentially detrimental manner. Simons and coworkers (1983) have suggested that hybridization of a small anti-sense transcript to E. coli Tn10 mRNA contributes to the regulation of transposition of that element. Stephenson and Zamecnik (1978) and Zamecnik and Stephenson (1978) have shown that synthetic oligodeoxynucleotides, complementary to Rous sarcoma virus terminal repeats, diminish normal viral infection and can inhibit viral RNA translation in vitro. However, these discoveries were not applied to the production of host resistance to a parasite.

Palukaitis et al. (1984) define cross-protection as a case where, the infection in a plant interferes directly with the replication of a second virus. These authors propose a model to explain the cross-protection phenomenon by plant viruses and viroids taking into account the observation that cross-protection requires that an inducing and a challenge virus (or viroid) encounter one another in the same cell.

Sequeira L. (1984) discusses the characteristics of cross-protection, its use for disease control and the mechanisms which have been proposed for its explaination. All said mechanisms involve a first infections by an intact inducing virus which promotes protection against a second challenging virus and imply the existence of a candidate gene for cross-protection, from a mild strain of the same virus.

Despite this fragmentary knowledge in the prior art, there still remains a need for a fully developed technique for producing resistance to parasites that is not based on the traditional methods of using a resistance gene from an immune strain of a host.

### Disclosure of the Invention

According, it is an object of this invention to provide a method of conferring resistance to a parasite to a host of the parasite which does not rely on the necessity of identifying and isolating a resistance gene from an immune strain of the host.

This and other objects of the invention as will hereinafter become pore readily apparent have been accomplished by providing a method for conferring resistance to a viral parasite to a plant or bacterial host of said parasite, which comprises:
a) isolating a viral nucleotide sequence coding for a viral protein, a modified viral protein or a viral protein component from said viral parasite, wherein the product of said viral nucleotide sequence is capable of disrupting infection of said host by said viral parasite;
b) inserting said viral nucleotide sequence or a DNA or RNA segment substantially homologous to at least a part of the viral nucleotide sequence or a DNA or RNA sequence functionally equivalent to the viral nucleotide sequence into the plant or bacterial host, by forming a recombinant DNA or RNA or functionally equivalent vector capable of integrating into the host chromosomes so that it stably replicates or is maintained as an episome ; and
c) expressing said viral nucleotide sequence, or said DNA or RNA sequence, in cells of said plant or bacterial host, whereby essential functions, which are under the control of the parasite's genes are disrupted by the presence of the expression product in the host which is dysfunctional, in excess, or appears in the wrong context or at the wrong developmental stage in the parasite's life cycle.

### Best Mode for Carrying Out the Invention

The concept of parasite-derived resistance is that host resistance to a particular parasite can effectively be engineered by introducing a gene, gene fragment, or modified gene or gene fragment of the pathogen into the host. This approach is based upon the fact that in any parasite-host interaction, there are certain parasite-encoded cellular functions (activities) that are essential to tile parasite but not to the host. An essential function is one which must operate if tile parasite is to survive or reproduce. These functions represent the Achilles heel of the parasite. If one of these functions is disrupted, the parasitic process will be stopped. "Disruption" refers to any change that diminishes the survival, reproduction, or infectivity of the parasite. Such essential functions, which are under the control of the parasite's genes, can lie disrupted by the presence of a corresponding gene product in the host which is (1) dysfunctional, (2) in excess, or (3) appears in the wrong context or at the wrong developmental stage in the parasite's life cycle. If such faulty signals are designed specifically for parasitic cell functions, they will have little effect on the host. Therefore, resistance to a particular pathogen can be achieved by cloning the appropriate parasite gene, if necessary modifying its expression, and transforming it into the host genome. By resistance is meant any reduction in virulence of the parasitic infection or any reduction in the susceptibility of the host to the parasite.

This approach to engineering resistance has important advantages:
1) The source of resistance genes would never be in question, since each parasite would bring with it the genes necessary for deriving resistance.
2) The stability of parasite-derived resistance will generally be greater than the stability of simply inherited forms of host resistance, for reasons that are discussed later in more detail.
3) The difficulties involved in cloning genes from host organisms, which generally have larger genomes relative to their pathogens, are lessened.
4) Parasite-derived resistance will have a minimal effect on the host and should not produce substances harmful to man.

The general concept of parasite-derived resistance has not previously been developed despite the prior art previously discussed. There are several reasons for this. Firstly, the examples of negative interaction have generally been serendipitous observations of a natural effect and have been viewed in isolation, as special cases. Secondly, negative interactions have generally been regarded from an academic perspective with little consideration for practical applications. This is most clearly evident in the above-mentioned papers on complimentary oligonucdeotides, wherein none of the authors make any connection with their own work and host/parasite relations or potential engineering of resistance. Also a recent review in a symposium on the use of anti-sense strands makes no mention of the application of this approach to host-parasite systems (Science, 1984, 225: 319). Thirdly, the general concept of parasite-derived resistance is based on a perspective other than the conventional wisdom. To the inventors' knowledge no one has deliberately engineered a parasite-derived resistance of any kind. Deliberateness is part of the claimed invention as indicated by language calling for "isolating" and "inserting" gene fragments.

In addition, a review of the literature has revealed an absence of any prior art in the following specific areas:
1) Use in the host of viral genes coding for regulatory proteins, such as coat proteins, to interfere with viral reproduction.
2) Use in the host of derivatives of a viral replicase gene or a viral reverse transcriptase gene for the purpose of interfering with viral reproduction.
3) Use of lysis genes derived from a parasite, to be used as inducible "self-destruct" genes for the engineering of hyper-sensitivity into the host.
4) Use in the host of avirulence genes from a parasite, for the purpose of over-riding the virulence genes in virulent strains of the parasite.
5) Use of parasite genes involved in the biosynthesis of key regulatory molecules, for the specific purpose of engineering the same biosynthetic capabilities into the host, such that the host produces hormones, neurotransmitters, pheromones, or similar molecules which are disruptive to the parasite.

As an example of how disease resistance can be engineered by this approach, the discussion below sets forth in detail how the genes of the bacteriophage Qβ can be used to make E. coli resistant to Q β infection. This example is not to be considered limiting of the invention but is an example of the ease with which the invention can be practiced, now that it is disclosed.

The biology of Q β and other RNA phages has been extensively documented (Zinder, 1975), and the cDNA sequence of its genome has been determined. The Q β genome has three major cistrons. These code for a maturation protein (involved in lysis and phage binding to host pili), a coat protein, and a subunit of the replicase enzyme. (A fourth gene product is a minor coat protein which is a read-through product of the coat cistron.)

The life cycle of Q β is basically as follows. The phage begins by binding to the sex pili of F' E. coli, through which it enters the cell and begins to translate its replicase subunit. Its replicase subunit polymerizes with three host subunits normally involved in host protein translation. The resulting hybrid tetrameric enzyme has RNA replicase activity specific for Q β. This specificity is due to the affinity between the Q β subunit of the tetrameric replicase and a short segment of the Q β genome within the replicase cistron. The replicase attaches to Q β RNA at this binding site and replicates the viral RNA. Late in the life cycle of Q β, coat protein and maturation protein accumulate in the host. The coat protein then binds to the replicase cistron and thereby represses translation of the replicase subunit. Termination of replication allows viral assembly, and eventually the maturation protein lyses the host, releasing a new population of infective Q β.

From a conventional (prior art) perspective, the life cycle of Q β suggests two potential mechanisms for developing resistance. Host-derived resistance might be developed by (1) blocking Q β binding to sex pili or (2) producing variant host subunits lacking affinity for the Q β replicase subunit. Blocking Q β binding is, in fact, a known mechanism for producing Q β resistance, since non-F' mutants lacking pili are immune to infection (Silverman, Rosenthal, Mobach and Valentine, 1968). However, this strategy clearly disrupts a mechanism which is relevant to the host's fitness as a species. The selection of variant forms of the host subunits which help make up the replicase enzyme may also be a naturally occuring mechanism conferring resistance. Since the host supplies 3 of the 4 subunits of the viral replicase, one might expect mutations within these genes to confer resistance. However, the extent to which these host subunits can be altered is clearly limited, since these subunits are essential to host protein synthesis and the survival of the host. Most of the variants of these host subunits would probably be lethal or sub-lethal for the host. Even non-lethal variants are likely to be suboptimal for protein translation efficiency. Therefore, both of the host-derived resistance mechanisms suggested by the Q β life cycle would be obtained at the expense of disrupting crucial host functions.

The prospect of being able to transfer genes from parasite to host provides a new approach to resistance. Viewed from this perspective, the life cycle of Q β suggests at least as many mechanisms of pathogen-derived resistance as host-derived resistance. Several strategies are seen to be promising : (1) deriving resistance from the Q β coat protein; and (2) deriving resistance from a modified Q β replicase.

Another strategy involving the maturation protein also appears feasible.

Resistance derived from the coat protein - The Q β coat protein is known to have a regulatory, as well as a structural role. Late in the phage life cycle, coat protein binds to and represses the cistron coding for the Q β replicase subunit, stopping replication and allowing viral assembly (Bernardi and Spahr, 1972). When cDNA to the coat protein translational sequence is linked to an E. coli promoter and introduced into E. coli, the coat protein is produced in the host. Expression of coat protein (in sufficient quantity) in the host will repress replication of any infecting Q β, thereby conferring resistance on the transformed host.

Resistance derived from a derivative of the replicase gene - The Q β replicase subunit has a dual affinity for a segment of the Q β genome and the three host replicase subunits (Kamen, 1970; Meyer, Webster and Weissmann, 1981). If the Q β replicase gene is cloned (as cDNA) and mutagenized, some variant forms will be able to bind to the Q β replicase site and at the same time fail to polymerize with the host subunits, a requirement to form a functional replicase. Alternatively, a portion of the replicase gene can be cloned to produce a polypeptide containing the functional domain for binding the replicase site but incapable of interacting with the host subunits. A transformed host producing such a modified replicase subunit would be Q β-resistant if the modified Q β replicase subunit or a portion of it binds to the replication sites of infecting Q β and effectively competes with native Q β replicase for binding sites, thus disrupting Q β replication.

Resistance derived from Q β Maturation Protein - Although the maturation protein's mode of action is not yet well understood (Karik and Billeter, 1983; Winter and Gold, 1983), it also represents a potential source of pathogen-derived resistance. A modified maturation protein in the host can block lysis. Alternatively, a repressed operon containing a wild-type maturation gene can be engineered in the host that would be activated by Q β infection. This would induce premature lysis of a host cell upon initial infection by Q β, constituting (on the population level) a form of hypersensitivity.

Although the examples set forth above describing methods by which bacteria can be protected from bacteria phage Q β are related in particular to a specific host/parasite system, the techiques are readily applicable to other systems, such as the protection of other organisms from both viral and non-viral infections. Techniques for achieving these results are set forth in more detail in the following paragraphs.

Virus resistance - The most likely early application of the concept of parasite-derived resistance is in engineering virus resistance. This is because the viral genome is small, and, since virus only propagates in the host, most of the genome is involved in pathenogenicity. Portions of the viral genome can be cloned and their potential for conferring resistance readily determined. Alternatively, resistance-conferring genes can be discovered empirically by testing the biological effect of various DNA restriction fragments of the viral genome. Most virus-derived resistances are likely to involve a block in replication. The methods described for engineering resistance to Q β are directly applicable to any virus which a) codes for a protein which helps regulate the virus' reproduction; b) has specific binding sites in its genome; c) synthesizes its own replicase or reverse transcriptase; or d) is bound by complementary reverse strand sequences of nucleic acid. In other words, these methods would apply to essentially all viruses.

While there has been some controversy among biochemists regarding whether plant viruses encode their own replicase, it now seems likely that most plant viruses do code for all or part of their replicases (Hall, Miller and Bujarski, 1982; Dorssers, Van der Meer, Kamen, Zabel, 1983). The first plant virus to have its replication mechanism characterized, turnip yellows mosaic virus, has proven analogous to Q β (Mouches, Candresse and Bove, 1984). This virus has been shown to to have a hybrid replicase, with its own sub-unit conferring specific binding to its genome. This indicates that the approach described for Q β replicase would also apply to this virus. It is likely that most or all RNA plant viruses will code either for their own replicase, a subunit of the replicase, or a protein modifying the specificity of the host's RNA polymerase. This means that the replicase-derived resistance strategy outlined for Q β will be directly applicable to a wide range of plant viruses. Many viruses have not yet been analyzed relative to this genetic structure. However, the very small size of the viral genome and the diversity of potential resistance mechanisms clearly indicates that a viral-derived resistance gene can be derived from any virus simply by using standard shotgun cloning methods and direct screening for subsequent resistance to the virus.

### Advantages and Limits of Pathogen-derived Resistance

Parasite-derived resistance represents a systematic and broadly-relevant approach to the problem of how to genetically engineer disease resistance. The rich possibilities of this approach are illustrated by the fact that three different strategies for deriving resistance from the Q β bacteriophage exist in a parasite having only three genes. There are several distinct advantages of parasite-derived resistance.

One of the most attractive features of parasite-derived resistance is that each new parasite or race of parasite that becomes a problem simultaneously brings with it the specific genes needed to engineer resistance to itself. These genes can be systematically identified within the parasite's genome. Once such genes have been identified, homologous genes in other parasite races or in related parasites will be readily identifiable by DNA hybridization techniques. This eliminates the need for repeated and exhaustive searches through the host's germplasm pools, seeking rare host resistance genes.

Another major advantage of this strategy is that it should not generally be disruptive of host functions. Van der Plank (1978), using evolutionary arguments and population genetics data, has argued that host genes controlling susceptibility exist because they involve essential host functions. Most hosts are genetically susceptible because the susceptible allele is optimal relative to its natural function. Host-derived resistance alleles, therefore, tend to disrupt the optimal functioning of the host. To the extent that this is true, most host-derived resistances attack the pathogen indirectly by replacing an optimal host gene product with a non-optimal host gene product which happens to be incompatible with the parasite. This is seen in the Q β system, where host-derived resistance is likely to be achieved either by disrupting sex pili formation or by tampering with the host's protein-synthesis machinery. A similar situation exists with sickle-cell anemia, which is harmful to humans when expressed but which confers resistance to malaria in persons who have both a recessive sickle-cell gene and a normal hemoglobin gene. The beauty of the concept of pathogen-derived resistance is that only pathogenic cell functions are attacked and are attacked directly, which will have minimal subsequent effect on the host. The specificity of parasite-derived resistance is not only desirable in terms of being non-disruptive to the host, but also of being non-harmful to man. Resistance based upon production of general toxicants, such as the natural pesticides of many resistant plant taxa, have been shown to be potentially harmful to man when ingested (Ames, 1983). The specificity of parasite-derived resistance should preclude, to a large extent, any such harm to man.

There are reasons to believe that parasite-derived resistance should be relatively durable compared to host-derived resistance. The ability of parasites to circumvent host-generated general toxicants is well known. Additionally, specific host-derived resistance genes are frequently overcome by matching gene-for-gene mutations to virulence in the parasite (Flor, 1971). In the case of host-derived Q β resistance, alterations in the host replicase sub-units (making them incompatible with the viral subunit, thereby conferring resistance), are easily matched by mutations in the Q β replicase subunit which restore subunit compatability, constituting a mutation to virulence. However, such gene-for-gene mutations circumventing resistance should be relatively rare in the case of parasite-derived resistance. In this case the parasite would usually be facing a new form of resistance, which it had not previously faced in its evolution. These types of resistances are likely to be very difficult for the parasite to overcome, especially where regulatory genes are involved. For example, if resistance to Q β was derived from the Q β coat protein gene, a new virulent Q β strain could only arise by first having a new binding site develop by mutation in the replicase cistron (without disrupting replicase function) which would not bind the native coat protein. Simultaneously a new coat protein would have to arise by mutation (without disrupting coat protein function) which would bind to the new binding site. Such a simultaneous and complementary set of mutations (which preserved both coat and replicase functions) should be extremely rare.

Last, engineering parasite-derived resistance should be considerably more approachable on the molecular level than engineering host-derived resistance. There are numerous reasons for this:
(1) this strategy would generally focus on the molecular biology of relatively simple organisms with short life cycles; (2) it would generally require only the identification and isolation of individual genes from small genomes; (3) unregulated, constitutive expression of the parasite-derived resistance genes would usually be effective; and (4) it would avoid the complex, multigenic biosynthetic pathways which are. the likely basis of many existing host-derived resistances.

There do not seen to be any obvious disadvantages to the parasite-derived approach to resistance, except that application of the strategy to non-virus parasites is only possible where mechanisms exist for macromolecular exchange between host and parasite. Most forms of parasitism, especially those forms displaying gene-for-gene resistance, allow ample opportunity for gene-product interactions and will be suitable for engineering parasite-derived resistance.

Techniques for the production of resistant host - As will be readily understood that those of ordinary skill in the art of genetic engineering, standard techniques of genetic engineering can readily be adopted to attain the goals set forth herein. Protection of a host against a virus, for example, can easily be achieved. Because of the reasons set forth above, it is not necessary to identify the gene being inserted into the host, although identification of the gene will make application of the method easier to perform. In general, genetic information (DNA or RNA) from any virus is isolated using standard procedures and cleaved into pieces of varying lengths, preferably containing at least 20 nucleotides if the DNA is to be transcribed in an anti-sense direction, or at least a functional portion and preferably an entire gene if the gene is to be expressed. DNA fragments are typically obtained using restriction endonuclease enzymes. The same enzyme (or enzymes) is then used to cleave a vector capable of replicating in the host or inserting into a host's chromosome. The vector can be a natural plasmid or transposon or any part thereof capable of replication in the host and, when desired, production of a gene product from the exogenous parasite gene fragment. Vectors derived from plasmids and other vectors normally present in the host are preferred. The viral DNA is inserted into the vector using standard techniques in either a sense direction (when expression of a gene product is desired) or an anti-sense direction. Proper tailoring of the gene fragment in the vector (e.g., employing appropriate 5' and 3' flanking sequences to ensure regulation, transcription, and translation as desired) is readily achieved using standard techniques, especially when simple constitutive expression is desired, as is suitable in most cases of parasite-derived resistance. As used in this application, the phrase "gene fragment" encompasses both entire genes, DNA segments that contain an entire gene or a portion thereof, and segments of DNA that are incomplete parts of a single gene. The word "gene" encompasses both DNA sequences that code for a peptide gene product and other DNA sequences that form a functional part of a chromosome or plasmid.

Although this specification generally refers to DNA alone when describing genetic information, vectors, or the like, this is done for ease of expression only. Any reference to DNA, unless clearly restricted to DNA and not to RNA, is equally applicable to RNA. For example, pathogenic RNA viruses can be the source of the parasite gene fragment, and non-virulent RNA viruses can act as vectors. In many instances, however, it is easier to work with DNA than RNA (e.g., more DNA restriction endonuclease enzymes are known), and use of cDNA prepared from RNA is a preferred embodiment of the invention when producing resistance to an RNA virus.

After a gene fragment has been isolated, the DNA sequence can be determined and modified, if desired, to produce similar DNA segments capable of being expressed as the same or similar gene products. For example, one or more codons can be replaced by equivalent codons to produce artificial DNA segments coding for the identical gene product. Alternately, a codon can be replaced by a codon that codes for a similar amino acid (e.g., a codon for lucine replaced by a codon for isoleucine or a codon for glutamic acid replaced by a codon for aspartic acid). Greater modification of the gene fragment is possible when a gene product of the parasite gene is being produced. For example, artificial DNA sequences containing a series of codons functionally equivalent (i.e., that code for the same amino acids) to the codon sequence in the parasite gene fragment are considered fully equivalent to the parasite gene fragment since they will produce the same gene product, even though the DNA sequence can be substantially different. Gene products not identical to the natural gene product but retaining the ability to produce a gene product capable of disrupting an essential activity of the parasite can be produced by systematic modification of codons (and thus the expressed gene products) followed by testing for parasite resistance. Such modified DNA segments must be substantially homologous to at least a part of the isolated gene fragment or a DNA sequence functionally equivalent thereto in order to be considered indicative of parasite-derived resistance. By "substantial homology" is meant at least 80%, preferably at least 90%, and most preferably at least 95% identity between the DNA sequence in question and the sequence to which it is being compared. Identical sequences are also covered by the same phrase. Comparisons for the purpose of determining homology are preferably made over a sequence of at least 15 and more preferably at least 21 nucleotides.

The phrase "isolating a gene fragment", as used in this application, refers to the process of obtaining a gene fragment to be used in the production of resistance in a useful form. The gene fragment does not have to be purified or otherwise separated from other cellular components, although this will occur in many processes. Instead, the word "isolated" is used to indicate that a gene has been obtained in a useful form by a deliberate process. For example, an "isolated gene fragment" can exist in a mixture of fragments from the DNA of a parasite that is to be used in a shotgun cloning procedure. A gene fragment is also still "isolated" when it is present in the form of a recombinant plasmid present in a bacterium being used in a shotgun cloning procedure to identify producers of desired parasite gene products (such as by use of monoclonal antibodies). Likewise, a segment of purified DNA comprising a parasite gene segment and termini from a cloning vector (e.g., obtained by cloning a parasite gene fragment in a bacterial plasmid prior to insertion into the final host) is also encompassed by this term. Other usable forms of gene fragments will be readily apparent to those skilled in genetic engineering.

Insertion of the parasite gene fragment into a host is readily achieved when the host is a bacterium or other unicellular organism since the major advances that have occurred recently in genetic engineering have generally involved insertion of vectors containing exogenous genes into unicellular hosts (especially bacteria and yeasts) and are directly applicable to the present method. "Insertion" encompasses any means of introducing genetic information into a host organism compatible with the limitations discussed in this specification. However, insertion in a manner to provide a heritable characteristic is preferred. In unicellar organisms this can readily be accomplished using heritable plasmids or by insertion of the parasite gene fragment into the host chromosome. These examples are not limiting, and other methods of inserting heritable genetic information, whether into unicellar or higher organisms, are equally applicable to the practice of this invention.

Proven methods for inserting new genes into higher organisms can now be found in a massive volume of current literature. There exist four basic methods of doing this (Baserga, Crose, and Povera, Eds., 1980) : (1) direct uptake of DNA or DNA-containing particles by the cell, (2) cell fusion with other cells or ghost cells, (3) microinjection, and (4) infective transforiation. A fifth method is being developed which involves the use of accelerated high-velocity one-micron-sized particles for the purpose of carrying DNA into cells and tissues.

Uptake mechanisms include the following: (1) induction of enhanced membrane permeability by use of Ca⁺⁺ and temperature shock (Mandel and Higa, 1970; Dityakin et al., 1972); (2) use of surface binding agents such as PEG (Chang and Cohen, 1979; Krens et al., 1982) or Ca(PO₄)₂ (Graham and van der Eb, 1973; Wigler et al., 1979); and (3) phagocytosis of particles such as liposomes (Uchimaya et al., 1982), organelles (Potrykus, 1973), or bacteria (Cocking, 1972), into the cell. These uptake mechanisms generally involve suspensions of single cells, where any existing cell wall materials have been removed enzymatically. Uptake protocols are generally quite simple and allow treatment of large numbers of cells en masse. In such systems most cells are unaffected, but cell selection procedures are available to recover the rare cells that have been transformed (Powers and Cocking, 1977).

Fusion mechanisms incorporate new genetic material into a cell by allowing it to fuse with another cell. A variation on this theme involves ghost cells. The membrane of killed cells are allowed to fill with a given DNA solution, such that cell fusion incorporates the DNA from the carrier "cell" into the living cell. Cell-to-cell fusion can be induced with the aid of such things as PEG (Bajaj, 1982) and Sendai virus particles (Uchida et al., 1980). As with uptake mechanisms, fusion technologies rely upon the use of single cell suspensions, where cells are enzymatically stripped of any cell wall material. While fusion technologies can have relatively good efficiencies in terms of numbers of cells affected, the problems of cell selection can be more complex, and the resulting cells are typically of elevated ploidy.

Microinjection technologies employ extremely fine, drawn out capillary tubes, which are called microelectrodes. These can be made sufficiently small that they can be used as syringe needles for the direct injection of biological substances into certain types of individual cells (Diacumakos, 1973; Graessmann and Graessmann, 1983). One modification of microinjection involves pricking with a solid-glass drawn needle, which carries in biological solutions which are bathing the cell (Yamomoto et al., 1981). Another modification is called ionophoresis (Purres, 1981; Ocho et al, 1981), which uses electrophoesis of substances out of the microelectrode and into the cell as an alternative to high pressure bulk flow. Microinjection procedures can give extremely high efficiencies relative to delivery into the cell. Because of this, microinjection has been used successfully in the transformation of individual egg cells.

In another example, foreign DNA was successfully injected into cotton pollen tubes without the pollen being damaged or its germination being inhibited. Although this involved a resistance gene from another plant instead of a parasite gene, the same technique can be used in the practice of the present invention. DNA was injected into the nucleus of cotton pollen grains germinating on cellophane using micro-manipulators and a micro-injection system. This operation was carried out on the fixed stage of an inverted research microscope equipped with Nomarski differential interference optics. Foreign DNA in a recipient nucleus was detected by epifluorescence after the incorporation of a fluorescent marker in the injected material. The DNA was introduced using "quickfill" tubing drawn to a tip diameter of 0.5 micron, and the DNA was injected into the nucleus iontophoretically. The germinating pollen was returned to the style where it continued to grow and fertilize the ovule. About 20 injections can be carried out per day. Seeds from the micro-injected plants were planted, and seedlings were raised and screened. Screening may be carried out by testing for the presence of the foreign gene by Southern blotting or for the presence of the gene product by means of enzyme inhibition assays. Other plants can be treated in the same manner.

Infective transformation employs non-injurious infective agents of the host, such as viruses, which naturally transmit part of their genome into the host. In plants, the principal mode of transformation now being practiced is the use of the infective agent Agrobacterium tumefaciens. This bacterium will naturally colonize cells of any dicotyledonous plant and transmit a specific "T-region" of its Ti-plasmid into the plant chromosome. Other plant vectors useful for the transformation of plants can similarly be used. Genes of interest can now be routinely engineered into the T-region and can be transmitted to the plant by the bacterium (see Fraley et al., 1983). Simple conincubation (growing plant cells_and bacterial cells together) has been shown to be extremely effective in transforming plant protoplasts and leaf disks, and whole transformed plants have now been regenerated in numerous plant species (see Horsch et al., 1984).

Parasites for use in use in this invention are viruses, whether the virus is a DNA or RNA virus.

Since a host is normally higher in the evolutionary scheme than the parasite, the term "host" does not encompass a virus, which resides at the bottom of the evolutionary scheme. However, any higher organism is capable of being infected by a parasite. The invention is readily applicable, for example, to bacteria grown in culture which need protection against infection from bacteriophages. Additionally, plants also can be readily protected from viruses using the method of the invention. Examples of hosts include bacteria, legumious plants (e.g., soybeans), cereal and forage crops (e.g., corn, wheat, rice and alfalfa) , food crops (e.g., tomatoes, potatoes, lettuce, and onions), ornamental plants (e.g., roses, junipers, and orchids), trees (e.g., pine, spruce, and walnut).

Examples of host/parasite systems in which either the host or the parasite is a unicellular organism (the most common situations) can be found in numerous microbiology textbooks and reference manuals, such as CRC Handbook of Microbiology, Condensed Edition, Laskin and Lechevalier (eds.), CRC Press, Cleveland, Ohio, 1974. Other examples of host/parasite systems are given below along with examples of how resistance to the parasite can be given to the host in that system. These examples are not limiting, and many other methods for achieving resistance are possible for each listed system.
1) There are a variety of bacteria important in industrial fermentation processes, such as Streptococcus lactis, Streptococens cremoris, and Lactobacillus species. During fermentation, infection by various bacteriophages is a common cause of fermentation failure. 'Bacterial resistance to such bacteriophage infection can be engineered by methods exactly analogous to the methods described above for engineering resistance to the Q β bacteriophage in E. coli.
2) There are hundreds of significant plant RNA viruses, and essentially all crop species are affected by one or more such viruses. Resistance to such viruses can be obtained in a manner closely analogous to Q β resistance in bacteria, by cloning fragments of the viruses into plant-transforming vectors such as a modified Ti-plasmid and transforming the appropriate plants. Plants transformed by various gene fragments can then be screened for resistance, using established plant breeding techniques. A few relevant viruses include alfalfa mosaic virus, brome mosaic virus, barley yellow dwarf virus, beet yellows virus, cucumber mosaic virus, lettuce necrotic yellows virus, maize chlorotic dwarf virus, pea enation virus, potato viruses S, X, and Y, southern bean mosaic virus, tomato ringspot virus, tobacco ringspot virus, tobacco mosaic virus, tobacco streak virus, turnip yellow mosaic virus, and wound tumor virus.

In addition to the above general procedures which can be used for preparing recombinant DNA molecules and transformed unicellular organisms in accordance with the practices of this invention, other known techniques and modifications thereof can be used in carrying out the practice of the invention. In particular, techniques relating to genetic engineering have recently undergone explosive growth and development. Many recent U.S. patents disclose plasmids, genetically engineered microorganisms, and methods of conducting genetic engineering which can be used in the practice of the present invention. For example, U.S. Patent 4,273,875 discloses a plasmid and a process of isolating the same. U.S. Patent 4,304,863 discloses a process for producing bacteria by genetic engineering in which a hybrid plasmid is constructed and used to transform a bacterial host. U.S. Patent 4,419,450 discloses a plasmid useful as a cloning vehicle in recombinant DNA work. U.S. Patent 4,362,867 discloses recombinant CDNA construction methods and hybrid nucleotides produced thereby which are useful in cloning processes. U.S. Patent 4,403,036 discloses genetic reagents for generating plasmids containing multiple copies of DNA segments. U.S. Patent 4,363,877 discloses recombinant DNA transfer vectors. U.S. Patent 4,356,270 discloses a recombinant DNA cloning vehicle and is a particularly useful disclosure for those with limited experience in the area of genetic engineering since it defines many of the terms used in genetic engineering and the basic processes used therein. U.S. Patent 4,336,336 discloses a fused gene and a method of making the same. U.S. Patent 4,349,629 discloses plasmid vectors and the production and use thereof. U.S. Patent 4,332,901 discloses a cloning vector useful in recombinant DNA. Although some of these patents are directed to the production of a particular gene product that is not within the scope of the present invention, the procedures described therein can easily be modified to the practice of the invention described in this specification by those skilled in the art of genetic engineering.

All of the patents and other publications cited in this specification are indicative of the level of skill and knowledge of those skilled in the arts to which the present invention pertains.

In addition to the method of producing resistance to a parasite described above in detail, this invention also encompasses host cells produced by the process of the invention as well as recombinant vectors and other products of genetic engineering useful in the practice of the invention.

The invention now being generally described, the same will be better understood by reference to certain specific examples which are included herein for purposes of illustration only and are not intended to be limiting of the invention or any embodiment thereof, unless so specified.

### EXAMPLE

The feasibility of the concept outlined above was proven with experiments using the bacteriophage Q β and its host, E. coli. Using cDNA clones of Q β ( Q β is an RNA phage) , plasmids were first constructed or obtained which would express part of the Q β cDNA in E. coli and confer resistance.
Coat Protein: The plasmid used for production of the coat protein was pGL101 obtained from R. B. Winter (Cell 33, 877). This plasmid expresses the coat protein under lac operator control, so its expression can be induced by IPTG (though there is also a constitutive expression). This plasmid as well as the others described below contain the gene encoding amp^{r}.
Testing for Resistance: The strain GM1 (provided by R. W. Webster) was transformed with pUR222 or one of the test plasmids described above. These strains were grown up, made competent, incubated with Q β and then plated out in soft agar. Plaque numbers and sizes were assessed to determine if resistance was taking place.

In an initial experiment to test the coat protein, GM1 + pUR222 and GM1 + pGL101 were grown in 10 mls L-broth containing ampicillin in IPTG. At stationary phase the cultures were pelleted and resuspended in 4 ml 50 mH YCaCl₂. A small portion, 0.1 ml, of this plating culture was incubated 60' with 10⁷ pfu of Q β. This was then plated on YT-AMP plates in 3 ml soft agar with IPG. The results were that the GM1 + pUR222 plates had thousands of plaques which soon (24 hrs) engulfed the plate; the GM1 + pGL101 plate at first showed no plaques but later developed many very small plaques.

To check the possibility that the GM1 strain + pGL101 resistance was due to loss of the F' element, the strains were subsequently grown on minimal medium lacking proline to maintain selection for the F'. The results are presented in Table 1. The coat protein plasmids could confer resistance to Qβ infection. This experiment was repeated twice with essentially the same results. After continued passage, however, the plasmid bearing Q β cDNA sequences rearranged or were lost. Additionally, the pGL101 was tested at higher titer (10¹¹pfu) ; it still conferred resistance. Coat-conferred resistance from the RNA phages f1 and f2 were tested. GM1 with pGL101 was resistant to f2 but not f1 as might be expected considering their modes of infection.

**Table 1**

| Strain + plasmid | | Inducer | # of Plaques | Size |
|---|---|---|---|---|
| | | | | |
| GM1 | - | + | 300 | normal |
| | puR222 | - | 360 | " |
| | " | + | 348 | " |
| | pGL101 | - | 176 | very small |
| | " | + | 101 | very very |
| small | | | | |

### References Cited

Ames, B.N. (1983). Science 221: 1256-1264.

Bajaj, Y.P.S. (1982). In: Reinert, J. and Bajaj, Y.P.S. (eds.), Plant Cell, Tissue and Organ Culture. Springer-Verlag, New York, pp. 467-496.

Baserga, R., C. Crose, G. Rovera, Eds. (1980). Introduction of macromolecules into viable mammalian cells. In: Sinstar Symposium Series VI. A.R. Liss Inc. New York.

Bernardi, A. and Spahr, P. (1972). Proc. Nat. Acad. Sci. 69: 3033.

Chang, S. and Cohen, S.N. (1979). Mol. Gen. Genet. 168: 111-115.

Cocking, E.C. (1972). Ann. Rev. Plant physiol. 23: 29-50.

Diacumakos, E.G. (1973). In: Prescott, D.M. (ed.), Methods in Cell Biology. Academic Press, new York, pp. 287-311.

Dityatkin, S.Y., Lisovskaya, K.V., Panzhava, N.N., Iliashenko, B.N. (1972). Biochimica et Biophysica Acta 281: 319-323.

Dorssers, L., Meer, J. van der, Kammen, A. van, and Zabel, P. (1983). Virology 125: 155-174.

Flashman, S.F. (1978). Mol. Gen. Genet. 166: 61-73

Flor, H.H. (1971). Ann. Rev. Phytopathol. 9: 27S-296.

Fraley, R.T., et al. (1983). Proc. Natl. Acad. Sci. 80: 4803-4807.

Gibbs, A. (1969). Adv. Virus Res. 14: 263-327.

Graessmann, M. and Graessmann, A. (1983). Methods in Enzymology 101: 482-492.

Graham, F.L. and van der Eb, A.J. (1973). Virology 52: 456.

Hall, T.C., W.A. Miller, and J.J. Bujarski. (1982). In: Advances in Plant pathology. P. 179. (Vol. 1) Academic Press, New York.

Hamilton, R.I. (1980). In: Viruses. Plant Disease: An Advanced Treatise. P. 279. (Vol. 5). Academic Press, NY.

Held, G.A., L.A. Bulla, E. Ferari, J. Hoch, A.I. Aronson, and S.A. Minnich (1982). Prov. Natl. Acad. Sci. 79: 6065-6069.

Horsch, R.B., R.T. Fraley, S.G. Rogers, P.R. Sanders, A. Lloyd, N. Hoffmann (1984). Science 223: 496-498.

Izant, J. and Weintraub, H. (1984). Cell 36: 1007-1015.

Kamen, R. (1970). Nature 228: 527-553.

Karnik, S. and Billeter, M.A. (1983). EMBO 2: 1521.

Krens, F.A., Molendijk, L., Wullems, G.J., and Schilperoort, R.A. (1982). Nature 296: 72.

Mandel, M. and Higa, A. (1970). J. Mol. Biol. 53: 159-162.

Marcus, P.I. and Carrier, D.H. (1967). J. Virology 1: 334.

Marcus, P.I. and Zuckerbraun, H.L. (1969). In: The Biology of Large RNA Viruses. P. 455. Acad. Press, New York.

Marcus, P.I. and Zuckerbraun, H.L. (1970). Ann N.Y. Acad. Sci. 173: 185-198.

Meyer, F., Weber, H. and Weissman, C. (1981). J. Mol. Biol. 153: 631-660.

Mizuno, T., Chou, M. and Inouye, M. (1984). Proc. Natl. Acad. Sci. USA 81: 1966-1970.

Mouches, C., Candresse, T. and Bove, J.M. (1984). Virology 134: 78-90.

Ocho, M., Nakai, S., Tasaka, K., Watanabe, S., and Oda, T. (1981). Acta Med. Okayama 35 (5): 381-384.

Potrykus, I. (1973). Z. Pflanzenphysiol. 70: 364-366.

Power, J.B. and Cocking, E.C. (1977). In: Reinhert, J., and Bajaj Y.P.S. (eds.) Plant Cell, Tissue and Organ Culture. Springer-Verlag, New York, pp. 497-505.

Purres, R.D. (1981). Academic Press, New York, 146 p.

Roberts, T.M., Kacich, R. and Ptashne, M. (1979). Proc. Natl. Acad. Sci. USA 76: 760-764.

Silverman, P.M., Rosenthal, S., Mobach, H., and Valentine, R.C. (1968). Virology 36: 142.

Uchida, T., Yamaizumi, M., Makada, E., Okada, Y. (1980). In: Introduction of macromolecules into viable mammalian cells. Windsor Symposium Series VI, A.R.-Liss Inc., New York, pp. 169-185.

Uchimiya, H., Ohgawara, T., and Harada, H. (1982). In: Fujiwara A. (ed.), Proc. 5th Intl. Cong. Plant Tissue and Cell Culture, Jap. Assoc. for Plant

Tissue Culture. Tokyo. pp. 507-508.

Wigler, M., Sweet, R., Sim, G.K., Wold, B., Pellicer, A., Lacy, E., Maniatis, T., Silverstein, S., and Axel, R. (1979). Cell 16: 777.

Winter, R.B. and Gold, L. (1983). Cell 33: 877-855.

Yamanoto, F., Furusawa, M., Furusawa, I., and Obinata, M. (1982). Exp. Cell Res. 142: 79-84.

Zinder, N.D. (ed) (1975). RNA Phages. Cold Springs Harbor Laboratory, NY pp. 428.

Barton, K.A., and Brill, W.J. (1983). Science 219: 671-676.

Illmensee, W. (ed.) (1984). Long Term Goals in Agricultural Plant Improvement. Genetic Manipulation - Impact on Man and Society. Cambridge Univ. Press.

Day, p.R., Barrett, J.A., and Wolfe, M.S. (1983). Evolution of host-parasite interaction. In:
Genetic Engineering of Plants: An Agricultural
Perspective. Kosuge, P., Meredith, C.P., and
Hollaender, A., eds. Plenum Press, New York. Polukaitis, P., Zaitlin, M. (1984). A Model to Explain the "Cross-Protection". Phenomenon Shown by Plant Viruses and Viroids. In: Plant-Microbe Interactions. Mc Millan Inc. pp 420-429 Sequeiza, L. (1984). Trends in Biotechnology, 2(2), pp 25-29

## Claims

1. A method for conferring resistance to a viral parasite to a plant or bacterial host of said parasite, which comprises:
a) isolating a viral nucleotide sequence coding for a viral protein, a modified viral protein or a viral protein component from said viral parasite, wherein the product of said viral nucleotide sequence is capable of disrupting infection of said host by said viral parasite;
b) inserting said viral nucleotide sequence or a DNA or RNA segment substantially homologous to at least a part of the viral nucleotide sequence or a DNA or RNA sequence functionally equivalent to the viral nucleotide sequence into the plant or bacterial host, by forming a recombinant DNA or RNA or functionally equivalent vector capable of integrating into the host chromosomes so that it stably replicates or is maintained as an episome; and
c) expressing said viral nucleotide sequence, or said DNA or RNA sequence, in cells of said plant or bacterial host, whereby essential functions, which are under the control of the parasite's genes, are disrupted by the presence of the expression product in the host which is dysfunctional, in excess or appears in the wrong context or at the wrong developmental stage in the parasite's life cycle.

2. The method of Claim 1, wherein, said inserting comprises transforming the plant or bacterial host with a vector carrying said nucleotide sequence where said vector is capable of stably replicating in the host or said nucleotide sequence is capable of being inserted in a chromosome of said host.

3. The method of Claim 2, wherein said host is a bacterium and said parasite is a virus capable of infecting said bacterium.

4. The method of Claim 2, wherein vector is a plasmid or transposon found in said host or a portion of said plasmid or transposon capable of functioning as a vector.

5. The method of Claim 2, wherein said host is a plant and said parasite is a virulent virus capable of infecting said plant.

6. The method of Claim 5, wherein said vector is a plasmid, transposable element or a non-injurious virus found in said host or a portion of said plasmid, transposable element, or virus capable of infecting said plant.

7. The method of Claim 5, wherein said vector is a Ti plasmid of Agrobacterium tumefaciens or a part thereof capable of functioning as a vector for the transformation of a plant.

8. The method of Claim 1, wherein said viral nucleotide sequence which is isolated, inserted and expressed in steps a), b) and c), respectively, is one coding for a modified viral protein or a viral protein component.

9. The method of claim 1 wherein the viral nucleotide sequence or a DNA or RNA segment substantially homologous to at least part of the viral nucleotide sequence or DNA or RNA sequence functionally equivalent to the viral nucleotide sequence which is inserted and expressed in step b) and c), respectively, is one codifying for a viral coat protein, or a modified viral replicase.

10. Cells of a plant or bacterial host which are resistant to a viral parasite of said plant or bacterial host wherein a viral nucleotide sequence, isolated from said viral parasite, coding for a viral protein, a modified viral protein or a viral protein component, the product of said viral nucleotide sequence being capable of disrupting infection of said host by said viral parasite, or a DNA or RNA segment substantially homologous to at least a part of said viral nucleotide sequence, or DNA or RNA sequence functionally equivalent to said viral nucleotide sequence, has been inserted by forming a recombinant DNA or RNA or functionally equivalent vector capable of integrating into the host chromosomes so that it stably replicates or is maintained as an episome, and said viral nucleotide sequence, or said DNA or RNA segment or sequence, is expressed, characterised in that the expression product of the inserted viral nucleotide sequence, or a DNA or RNA segment substantially homologous to at least part of said viral nucleotide sequence, or a DNA or RNA sequence functionally equivalent to said viral nucleotide sequence, disrupts essential functions, which are under the control of the parasite's genes, in that it is dysfunctional, in excess or appears in the wrong context or at the wrong developmental stage in the parasite's life cycle.

11. Cells of a plant or bacterial host of claim 10 wherein the viral nucleotide sequence or a DNA or RNA segment substantially homologous to at least part of the viral nucleotide sequence or a DNA or RNA sequence functionally equivalent to the viral nucleotide sequence, which has been inserted and is expressed in said host, is one codifying for a viral coat protein or a modified viral replicase.

12. A plant which is resistant to a viral parasite of said plant wherein a viral nucleotide sequence, isolated from said viral parasite, coding for a viral protein, a modified viral protein or a viral protein component, the product of said viral nucleotide sequence being capable of disrupting infection of said plant by said viral parasite, or a DNA or RNA segment substantially homologous to at least part of said viral nucleotide sequence, or a DNA or RNA sequence functionally equivalent to said viral nucleotide sequence, has been inserted by forming a recombinant DNA or RNA or functionally equivalent vector capable of integrating into the plant chromosomes so that it stably replicates or is maintained as an episome, and said viral nucleotide sequence, or said DNA or RNA segment or sequence, is expressed, characterised in that the expression product of the inserted viral nucleotide sequence, or DNA or RNA segment substantially homologous to at least part of said viral nucleotide sequence, or DNA or RNA sequence functionally equivalent to said viral nucleotide sequence, disrupts essential functions, which are under the control of the parasite's genes, in that it is dysfunctional, in excess or appears in the wrong context or at the wrong developmental stage in the parasite's life cycle.

13. A plant of claim 12 wherein the viral nucleotide sequence, or a DNA or RNA segment substantially homologous to at least part of the viral nucleotide sequence or a DNA or RNA sequence functionally equivalent to the viral nucleotide sequence, which has been inserted and is expressed in said plant, is one codifying for a viral coat protein or a modified viral replicase.

## Patentansprüche

1. Verfahren, um einem pflanzlichen oder bakteriellen Wirt eines viralen Parasiten Resistenz gegenüber diesem viralen Parasiten zu verleihen, umfassend die folgenden Stufen:
a) Isolierung einer viralen Nukleotidsequenz, die für ein virales Protein, ein modifiziertes virales Protein oder eine virale Proteinkomponente des genannten viralen Parasiten codiert, wobei das Produkt der viralen Nukleotidsequenz in der Lage ist, die Infektion des Wirts durch den viralen Parasiten zu zerstören;
b) Insertion der viralen Nukleotidsequenz oder eines DNA- oder RNA-Segments, das im wesentlichen homolog zu wenigstens einem Teil der viralen Nukleotidsequenz ist, oder einer DNA- oder RNA-Sequenz, die funktionell äquivalent zu der viralen codierenden Sequenz ist, in den pflanzlichen oder bakteriellen Wirt, durch Erzeugung einer rekombinanten DNA oder RNA oder eines funktionell äquivalenten Vektors, der in der Lage ist, in die Wirtschromosomen zu integrieren, so dass er in stabiler Weise repliziert oder als Episom erhalten bleibt; und
c) Expression der viralen Nukleotidsequenz oder der DNA- oder RNA-Sequenz in Zellen des pflanzlichen oder bakteriellen Wirts, wobei essentielle Funktionen, die unter der Kontrolle der Parasitengene stehen, durch das Vorhandensein des Expressionsproduktes im Wirt zerstört werden, wobei das Expressionsprodukt dysfunktional ist, im Überschuss vorliegt oder in falschem Kontext erscheint oder in einer falschen Entwicklungsstufe im Lebenszyklus des Parasiten erscheint.

2. Verfahren nach Anspruch 1, dadurch **gekennzeichnet,** dass die Insertion die Transformation des pflanzlichen oder bakteriellen Wirts mit einem Vektor, der die Nukleotidsequenz enthält, umfasst, wobei der Vektor in der Lage ist, in stabiler Weise im Wirt zu replizieren, oder die Nukleotidsequenz in der Lage ist, in das Chromosom des Wirts zu insertieren.

3. Verfahren nach Anspruch 2, dadurch **gekennzeichnet,** dass der Wirt ein Bakterium ist und der Parasit ein Virus ist, das in der Lage ist, das Bakterium zu infizieren.

4. Verfahren nach Anspruch 2, dadurch **gekennzeichnet,** dass der Vektor ein Plasmid oder Transposon, das in dem Wirt gefunden wird, oder ein Teil des Plasmids oder Transposons, das in der Lage ist, als Vektor zu wirken, ist.

5. Verfahren nach Anspruch 2, dadurch **gekennzeichnet,** dass der Wirt eine Pflanze ist und dass der Parasit ein virulentes Virus ist, das in der Lage ist, die Pflanze zu infizieren.

6. Verfahren nach Anspruch 5, dadurch **gekennzeichnet**, dass der Vektor ein Plasmid, ein transponierbares Element oder ein nicht-schädliches Virus ist, das in dem Wirt gefunden wird, oder ein Teil des Plasmids, transponierbaren Elementes oder Virus, das in der Lage ist, die Pflanze zu infizieren.

7. Verfahren nach Anspruch 5, dadurch **gekennzeichnet**, dass der Vektor ein Ti-Plasmid von Agrobacterium tumefaciens oder ein Teil davon, der in der Lage ist, als Vektor für die Transformation einer Pflanze zu wirken, ist.

8. Verfahren nach Anspruch 1, dadurch **gekennzeichnet**, dass die virale Nukleotidsequenz, die in den Stufen a), b) bzw. c) isoliert, insertiert bzw. exprimiert wird, eine Sequenz ist, die für ein modifiziertes virales Protein oder eine virale Proteinkomponente codiert.

9. Verfahren nach Anspruch 1, dadurch **gekennzeichnet,** dass die virale Nukleotidsequenz oder ein DNA- oder ein RNA-Segment, das im wesentlichen homolog zu wenigstens einem Teil der viralen Nukleotidseguenz ist, oder eine DNA- oder RNA-Sequenz, die funktionell äquivalent zu der viralen Nukleotidsequenz ist, die in den Stufen b) bzw. c) insertiert bzw. exprimiert wird, eine Sequenz ist, die für ein virales Hüllprotein oder eine modifizierte virale Replikase codiert.

10. Zellen eines pflanzlichen oder bakteriellen Wirts, die gegenüber einem viralen Parasiten des pflanzlichen oder bakteriellen Wirts resistent sind, wobei eine virale Nukleotidsequenz, die aus dem viralen Parasiten isoliert wurde und die für ein virales Protein, ein modifiziertes virales Protein oder einen viralen Proteinbestandteil codiert, wobei das Produkt der viralen Nukleotidsequenz in der Lage ist, die Infektion des Wirts durch den viralen Parasiten zu zerstören, oder ein DNA- oder RNA-Segment, das im wesentlichen homolog zu wenigstens einem Teil der genannten viralen Nukleotidsequenz ist, oder eine DNA- oder RNA-Sequenz, die funktionell zu der viralen Nukleotidsequenz äquivalent ist, unter Erzeugung einer rekombinanten DNA oder RNA oder eines funktionell äquivalenten Vektors, der in der Lage ist, in das Wirtschromosom zu integrieren, insertiert wurde, so dass er in stabiler Weise repliziert oder als Episom aufrechterhalten wird und die virale Nukleotidsequenz oder das DNA- oder RNA-Segment oder die DNA- oder RNA-Sequenz exprimiert wird, dadurch **gekennzeichnet** , dass das Expressionsprodukt der insertierten viralen Nukleotidsequenz oder eines DNA- oder RNA-Segments, das im wesentlichen homolog zu wenigstens einem Teil der viralen Nukleotidsequenz ist, oder einer DNA- oder RNA-Sequenz, die im wesentlichen äquivalent zu der viralen Nukleotidsequenz ist, essentielle Funktionen zerstört, die unter der Kontrolle der Gene des Parasiten stehen, wobei das Expressionsprodukt dysfunktional ist, im Überschuss vorliegt oder im falschen Kontext oder in einer falschen Entwicklungsstufe im Lebenszyklus des Parasiten erscheint.

11. Zellen eines pflanzlichen oder bakteriellen Wirts nach Anspruch 10, dadurch **gekennzeichnet**, dass die virale Nukleotidsequenz oder ein DNA- oder RNA-Segment, das im wesentlichen homolog zu wenigstens einem Teil der viralen Nukleotidsequenz ist, oder eine DNA- oder RNA-Sequenz, die im wesentlichen äquivalent zu der viralen Nukleotidsequenz, die in den Wirt insertiert wurde und in diesen exprimiert wird, ist, eine Sequenz ist, die für ein virales Hüllprotein oder eine modifizierte virale Replikase codiert.

12. Pflanze, die gegenüber einem viralen Parasiten der Pflanze resistenz ist, wobei eine virale Nukleotidsequenz, die von dem viralen Parasiten isoliert wurde und die für ein virales Protein, ein modifiziertes virales Protein oder eine virale Proteinkomponente codiert, wobei das Produkt der viralen Nukleotidsequenz in der Lage ist, die Infektion der Pflanze durch den viralen Parasiten zu zerstören oder ein DNA- oder RNA-Segment im wesentlichen homolog zu wenigstens einem Teil der viralen Nukleotidsequenz ist oder eine DNA- oder RNA-Sequenz, die funktionell äquivalent zu der viralen Nukleotidsequenz ist, unter Bildung einer rekombinanten DNA oder RNA oder eines funktionell äquivalenten Vektors, der in der Lage ist, in das Pflanzenchromosom so zu integrieren, dass er in stabiler Weise repliziert oder als Episom aufrechterhalten wird, insertiert wurde und die virale Nukleotidsequenz oder das DNA- oder RNA-Segment oder die DNA- oder RNA-Sequenz exprimiert wird, dadurch **gekennzeichnet**, dass das Expressionsprodukt der insertierten viralen Nukleotidsequenz oder das DNA- oder RNA-Segment, das im wesentlichen homolog zu wenigstens einem Teil der viralen Nukleotidsequenz ist, oder die DNA- oder RNA-Sequenz, die funktionell äquivalent zu der viralen Nukleotidsequenz ist, essentielle Funktionen zerstört, die unter Kontrolle der Gene des Parasiten stehen, wobei das Expressionsprodukt dysfunktional ist, im Überschuss vorliegt, im falschen Kontext erscheint oder zu einer falschen Entwicklungsstufe im Lebenszyklus des Parasiten erscheint.

13. Pflanze nach Anspruch 12, dadurch **gekennzeichnet,** dass die virale Nukleotidsequenz oder ein DNA- oder RNA-Segment, das im wesentlichen homolog zu wenigstens einem Teil der viralen Nukleotidsequenz ist, oder eine DNA- oder RNA-Sequenz, die funktionell äquivalent zu der viralen Nukleotidsequenz ist, die insertiert wurde und in der Pflanze exprimiert wird, eine Sequenz ist, die für ein virales Hüllprotein oder eine modifizierte virale Replikase codiert.

## Revendications

1. Procédé pour conférer une résistance vis-à-vis d'un parasite viral à un hôte végétal ou bactérien dudit parasite, qui comprend :
a) l'isolation d'une séquence de nucléotides virale codant une protéine virale, une protéine virale modifiée ou un composant de protéine virale provenant dudit parasite viral, où le produit de ladite séquence de nucléotides virale est capable de rompre l'infection dudit hôte par ledit parasite viral ;
b) l'insertion de ladite séquence de nucléotides virale ou d'un segment d'ADN ou d'ARN pratiquement homologue d'au moins une partie de la séquence de nucléotides virale ou d'une séquence d'ADN ou d'ARN fonctionnellement équivalente à la séquence de nucléotides virale dans l'hôte végétal ou bactérien, par formation d'un ADN ou ARN recombiné ou d'un vecteur fonctionnement équivalent capable de s'intégrer dans les chromosomes de l'hôte de façon qu'il se réplique de façon stable ou soit maintenu sous forme d'un épisome ; et
c) l'expression de ladite séquence de nucléotides virale ou de ladite séquence d'ADN ou d'ARN dans des cellules dudit hôte végétal ou bactérien, de façon que des fonctions essentielles, qui sont sous le contrôle des gènes du parasite, soient rompues par la présence du produit d'expression dans l'hôte qui est dysfonctionnel, en excès, ou apparaît dans le contexte erroné ou au niveau du stade développemental erroné dans le cycle de vie du parasite.

2. Procédé selon la revendication 1, dans lequel ladite insertion comprend la transformation de l'hôte végétal ou bactérien avec un vecteur portant ladite séquence de nucléotides, où ledit vecteur est capable de se répliquer de façon stable dans l'hôte ou ladite séquence de nucléotides est capable d'être insérée dans un chromosome dudit hôte.

3. Procédé selon la revendication 2, dans lequel ledit hôte est une bactérie et ledit parasite est un virus capable d'infecter ladite bactérie.

4. Procédé selon la revendication 2, dans lequel ledit vecteur est un plasmide ou transposon trouvé dans ledit hôte ou une partie dudit plasmide ou transposon capable de fonctionner comme un vecteur.

5. Procédé selon la revendication 2, dans lequel ledit hôte est une plante et ledit parasite est un virus virulent capable d'infecter ladite plante.

6. Procédé selon la revendication 5, dans lequel ledit vecteur est un plasmide, un élément transposable ou un virus non nocif trouvé dans ledit hôte ou une partie dudit plasmide, élément transposable, ou virus capable d'infecter ladite plante.

7. Procédé selon la revendication 5, dans lequel ledit vecteur est un plasmide Ti de Agrobacterium tumefaciens ou une partie de celui-ci capable de fonctionner comme un vecteur pour la transformation d'une plante.

8. Procédé selon la revendication 1, dans lequel ladite séquence de nucléotides virale qui est isolée, insérée et exprimée dans les étapes a), b) et c), respectivement, est une séquence codant une protéine virale modifiée ou un composant de protéine virale.

9. Procédé selon la revendication 1, dans lequel la séquence de nucléotides virale ou un segment d'ADN ou d'ARN pratiquement homologue d'au moins une partie de la séquence de nucléotides virale ou de la séquence d'ADN ou d'ARN fonctionnellement équivalente à la séquence de nucléotides virale qui est insérée et exprimée dans les étapes b) et c), respectivement, est une telle séquence codant une protéine d'enrobage virale ou une réplicase virale modifiée.

10. Cellules d'un hôte végétal ou bactérien qui sont résistantes à un parasite viral dudit hôte végétal ou bactérien, dans lesquelles une séquence de nucléotides virale, isolée à partir dudit parasite viral, codant une protéine virale, une protéine virale modifiée ou un composant de protéine virale, le produit de ladite séquence de nucléotides virale étant capable de rompre une infection dudit hôte par ledit parasite viral, ou un segment d'ADN ou d'ARN pratiquement homologue d'au moins une partie de ladite séquence de nucléotides virale, ou une séquence d'ADN ou d'ARN fonctionnellement équivalente à ladite séquence de nucléotides virale, a été insérée par formation d'un ADN ou ARN recombiné ou d'un vecteur fonctionnellement équivalent capable de s'intégrer dans les chromosomes de l'hôte de façon qu'il se réplique de façon stable ou soit maintenu sous forme d'un épisome, et ladite séquence de nucléotides virale, ou ladite séquence ou segment d'ADN ou d'ARN, est exprimée, caractérisées en ce que le produit d'expression de la séquence de nucléotides virale insérée, ou d'un segment d'ADN ou d'ARN pratiquement homologue d'au moins une partie de ladite séquence de nucléotides virale, ou d'une séquence d'ADN ou d'ARN fonctionnellement équivalente à ladite séquence de nucléotides virale, rompt des fonctions essentielles, qui sont sous le contrôle des gènes du parasite, et en ce que cette séquence est dysfonctionnelle, en excès ou apparaît dans un contexte erroné ou dans un stade développemental erroné dans le cycle de vie du parasite.

11. Cellules d'un hôte végétal ou bactérien selon la revendication 10, dans lesquelles la séquence de nucléotides virale ou un segment d'ADN ou d'ARN pratiquement homologue d'au moins une partie de la séquence de nucléotides virale ou une séquence d'ADN ou d'ARN fonctionnellement équivalente à la séquence de nucléotides virale, qui a été insérée et est exprimée dans ledit hôte, est une telle séquence codant une protéine d'enrobage virale ou une réplicase virale modifiée.

12. Plante qui est résistante à un parasite viral de ladite plante, dans laquelle une séquence de nucléotides virale, isolée à partir dudit parasite viral, codant une protéine virale, une protéine virale modifiée ou un composant de protéine virale, le produit de ladite séquence de nucléotides virale étant capable de rompre une infection de ladite plante par ledit parasite viral, ou un segment d'ADN ou d'ARN pratiquement homologue d'au moins une partie de ladite séquence de nucléotides virale, ou une séquence d'ADN ou d'ARN fonctionnellement équivalente à ladite séquence de nucléotides virale, a été insérée par formation d'un ADN ou ARN recombiné ou d'un vecteur fonctionnellement équivalent capable de s'intégrer dans les chromosomes de la plante de façon qu'il se réplique de façon stable ou soit maintenu sous forme d'un épisome, et ladite séquence de nucléotides virale, ou ladite séquence ou segment d'ADN ou d'ARN, est exprimée, caractérisée en ce que le produit d'expression de la séquence de nucléotides virale insérée, ou d'un segment d'ADN ou d'ARN pratiquement homologue d'au moins une partie de ladite séquence de nucléotides virale, ou d'une séquence d'ADN ou d'ARN fonctionnellement équivalente à ladite séquence de nucléotides virale, rompt des fonctions essentielles, qui sont sous le contrôle des gènes du parasite, et en ce que cette séquence est dysfonctionnelle, en excès ou apparaît dans un contexte erroné ou dans un stade développemental erroné dans le cycle de vie du parasite.

13. Plante selon la revendication 12, dans laquelle la séquence de nucléotides virale ou un segment d'ADN ou d'ARN pratiquement homologue d'au moins une partie de la séquence de nucléotides virale ou une séquence d'ADN ou d'ARN fonctionnellement équivalente à la séquence de nucléotides virale, qui a été insérée et est exprimée dans ladite plante, est une telle séquence codant une protéine d'enrobage virale ou une réplicase virale modifiée.
